# EUROPEAN PATENT APPLICATION

(11) **EP 1 532 979 A1**
(43) Date of publication of application: **25.05.2005**
(21) Application number: 03761815.4
(22) Date of filing: 26.06.2003
(51) Int. Cl.: A61K 31/4035, A61P 3/10, A61P 43/00

(54) **DRUG COMPOSITION FOR BLOOD SUGAR CONTROL**

(30) Priority: 28.06.2002 JP 2002189556
(71) Applicant: Kissei Pharmaceutical Co., Ltd., Matsumoto-shi Nagano 399-8710 (JP)
(72) Inventor: MIKOSHIBA, Imao c/o Tokyo Head Office, Bunkyo-ku, Tokyo 112-0002 (JP); SUZUKI, Hisao, Minamiazumi-gun, Nagano 399-8101 (JP); KIYONO, Yuji c/o Tokyo Head Office, Bunkyo-ku, Tokyo 112-0002 (JP)
(74) Representative: Hayes, Adrian Chetwynd
(86) International application number: PCT/JP2003/008083
(87) International publication number: WO 2004/002473

(57) **Abstract**

The present invention provides pharmaceutical compositions which can achieve good state of glycemic control and correct postprandial hyperglycemia and early morning fasting hyperglycemia. The present pharmaceutical composition is for administration before meal to control blood glucose, which comprises 5 to 45 mg, as a single dose, of mitiglinide or a pharmaceutically acceptable salt thereof, or a hydrate thereof (for example, mitiglinide calcium salt hydrate). And said compositions are extremely useful for prevention or treatment of, for example, type II diabetes, because the frequency of adverse drug reactions such as hypoglycemic symptoms and gastrointestinal disorders is low.

## Description

### Technical Field

The present invention relates to a pharmaceutical composition for glycemic control of a type II diabetic patient which contains mitiglinide or a pharmaceutically acceptable salt thereof, or a hydrate thereof and which is prepared as a pharmaceutical composition to be taken before meals, and a method of uses thereof. The present invention also relates to a method for glycemic control of a type II diabetic patient, which comprises of administrating mitiglinide or a pharmaceutically acceptable salt thereof, or a hydrate thereof before meals, and to a use of mitiglinide or a pharmaceutically acceptable salt thereof, or a hydrate thereof for the manufacture of a pharmaceutical composition for glycemic control of a type II diabetic patient.

### Background Art

Diabetes is classified briefly into type I diabetes, type II diabetes, diabetes due to other certain mechanism or disorders, and gestational diabetes mellitus. Type I diabetes used to be called juvenile-onset diabetes or insulin dependent diabetes mellitus (IDDM), and type II diabetes used to be called adult-onset type diabetes mellitus or noninsulin dependent diabetes mellitus (NIDDM).

Diabetes is diagnosed in a patient when any of the following results is confirmed at both of twice examinations held on different days: 1) casual plasma glucose is not less than 200 mg/dL, 2) early morning fasting plasma glucose (FPG) is not less than 126 mg/dL, or 3) 2 hour value of 75 g oral glucose tolerance test is not less than 200 mg/dL. In a case that HbA_{1C} value is not less than 6.5%, diabetes is determined in a patient when the result meets any above-mentioned criterion even in one-time examination (see Reference 1).

Glycemic control is set up as a target for treatment of these diabetic patients, and the purposes are to maintain their quality of dairy life (QOL) like healthy people and to ensure their lives like healthy people by maintaining their good state of glycemic control, and furthermore, to prevent development and progression of diabetic microvascular complications (diabetic retinopathy, diabetic nephropathy, diabetic neuropathy and the like) and arteriosclerotic diseases (ischemic heart disease, cerebrovascular disease, arteriosclerosis obliterans and the like). HbA_{1C} value is used as a primary indication, and the targeted value is preferably not more than 7% and more preferably less than 6.5%. In addition, a 2 hour value of postprandial plasma glucose and a fasting plasma glucose are used as supportive indications of HbA_{1C} value. Two hundred (200) mg/dL for 2 hour value of postprandial plasma glucose and 100 to 140 mg/dL for fasting plasma glucose are targeted, respectively (see References 2 and 3).

In a recent large-scale clinical study in the UK on type II diabetes, the importance of glycemic control for treatment of diabetes has been confirmed. For example, 0.9% decrease in HbA_{1C} value caused 10% reduction of diabetes-associated mortality. And, it has been reported that the occurrence of cardiac infarction and microvascular complication notably decreased by 16% and 25%, respectively, and that provides good effects on development and progression of diabetic complications (see Reference 4). Furthermore, it has been reported that overt diabetic nephropathy is increasingly frequent with HbA_{1C} value over 7.5% and diabetic retinopathy occurs in high frequency in cases with fasting plasma glucose of 140 mg/dL or more.

As mentioned above, glycemic control is important for treatment of diabetic patients, and in order to maintain a good state of glycemic control, it is necessary to administrate appropriate doses in adequate usages under careful administration plans depending on the types, activities, dispositions and the like of used drugs. In addition, the points to pay attention in glycemic control are not causing any prolonged hypoglycemia and steadily controlling intraday blood glucose level including postprandial and fasting blood glucose.

Mitiglinide calcium salt hydrate (the chemical name: (+)-monocalcium bis[(2S, 3a, 7a-*cis*)-α-benzylhexahydro-γ-oxo-2-isoindolinebutyrate] dihydrate) is a rapid- and short-acting insulin secretagogue having the following chemical structure, and known as a compound expected as an agent to correct a postprandial hyperglycemic state. However, anything has not been reported on the disposition, the methods of use for glycemic control or the like of mitiglinide.

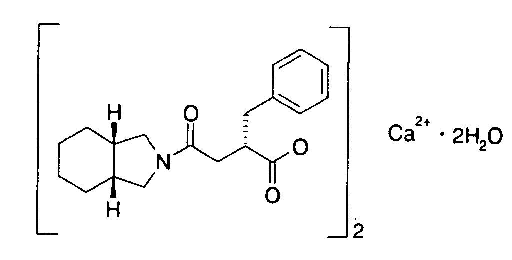

In addition, there is a report on a immediate release formulation which comprises mitiglinide calcium salt hydrate as an active ingredient. However, it is just an immediate release formulation, which is not prescribed only based on the disposition of mitiglinide but also the use for glycemic control.
Reference 1: [The guide for treatment of diabetes 2002-2003], edited by The Japan Diabetes Society, Edition 1, Bunkodo Inc., May 9, 2002, p.14-15;
Reference 2: [The guide for treatment of diabetes 2002-2003], edited by The Japan Diabetes Society, Edition 1, Bynkodo Inc., May 9, 2002, p.18-19;
Reference 3: [Today's treatment drugs, Explanations and Handbook (Konnichi-no-chiryoyaku Kaisetsu-to-binran)], edited by Yu Mizushima , Edition 24, Nankodo Inc. , March 15, 2002, p.297;
Reference 4: [Lancet], September 12, 1998, Vol.352, No.9131, p.837-853;
Reference 5: Japan Patent Publication No.356459/1992
Reference 6: International Patent Publication No.2000/71117 pamphlet.

### Disclosure of the Invention

The present inventors have studied earnestly on the activities and disposition of mitiglinide or pharmaceutically acceptable salts thereof, or hydrates thereof, and established an appropriate dosage and usage. Using a pharmaceutical composition prepared based on the findings obtained those studies, the inventors conducted a clinical study as described below. As a result, it was found that by administrating mitiglinide calcium salt hydrate in a manner as described below, an excellent glycemic control is achieved and postprandial hyperglycemia is efficiently inhibited, furthermore, early morning fasting hyperglycemia is inhibited, and the frequencies of concerned hypoglycemic symptoms and gastrointestinal disorders are low, and that said dosage and usage are extremely effective to prevent and treat type II diabetes, and thereby the present invention has been completed.

The present invention is to provide an excellent pharmaceutical composition for glycemic control of a type II diabetic patient and a method of use the same. Moreover, the present invention is to provide a preferable method of use of the pharmaceutical composition for type II diabetic patients.

For more details, the present inventors found that the required dosage of mitiglinide or a pharmaceutically acceptable salt thereof, or hydrate thereof to reduce HbA_{1C} value significantly is 5 mg and more as a single dose and that the half-life in disposition is of the said dose about 1.5 hour. Based on the findings, the inventors evaluated an appropriate dosage and usage, and as a result, it was found that by administrating 5 to 45 mg, or preferably 5 to 22 mg of mitiglinide calcium salt hydrate three times a day before each meal (within 10 minutes before starting meal), preferably just before meal (within 5 minutes before starting meal), for 4 weeks or more, the HbA_{1C} value significantly decrease and the glycemic control can be improved, and in addition, the frequencies of hypoglycemic symptoms and gastrointestinal disorders such as an increase of abdominal wind are low. Moreover, it was found that an increase of postprandial blood glucose level are markedly suppressed, an excellent hypoglycemic action is exerted even after 2 hours after meal, and in addition, early morning fasting plasma glucose is significantly suppressed. The present invention is based on these findings.

That is, the present invention relates to a pharmaceutical composition for glycemic control of a type II diabetic patient, which is prepared for administration before meal and comprises 5 to 45 mg of mitiglinide or a pharmaceutically acceptable salt thereof, or a hydrate thereof as a single dose, and relates to a use of the pharmaceutical composition for glycemic control of a type II diabetic patient and for prevention or treatment of type II diabetes.

The present invention also relates to a method for glycemic control and a method for improvement of postprandial hyperglycemia in a type II diabetic patient, which comprises administrating before meal 5 to 45 mg of mitiglinide or a pharmaceutically acceptable salt thereof, or a hydrate thereof as a single dose.

Moreover, the present invention relates to a use of mitiglinide or a pharmaceutically acceptable salt thereof, or a hydrate thereof for the manufacture of the above pharmaceutical composition for administration before meal to control blood glucose in a type II diabetic patient.

Further details are described about the present invention below.

In the present invention, the term of "postprandial hyperglycemia" means that a 1 hour value and/or a 2 hour value of postprandial plasma glucose (PPG) are not less than 200 mg/dL including that a casual plasma glucose level or 2 hour level of a 75 g oral glucose tolerance test is not less than 200 mg/dL. In addition, the term of "fasting hyperglycemia" means that early morning fasting plasma glucose (FPG) is not less than 126 mg/dL.

The target patients in the present invention are type II diabetic patients, especially type II diabetic patients who require to control their blood sugar. As a more applicable case, a patient with postprandial hyperglycemia is illustrated, and a patient with postprandial hyperglycemia accompanied with fasting hyperglycemia can be also illustrated. As a pharmaceutically acceptable salt of mitiglinide, an salt with an inorganic base such as a sodium salt, a potassium salt, a calcium salt and the like, a salt with an organic amine or an amino acid such as morpholine, piperidine, phenylalanine, and the like can be illustrated, and a calcium salt is preferable. In addition, as an active ingredient in the present invention, mitiglinide calcium salt hydrate is the most preferable. To maintain the good state of glycemic control, orally administrating 5 to 45 mg as a single dose of mitiglinide or a pharmaceutically acceptable salt thereof, or a hydrate thereof is preferable, and by administration in such a manner, not only glycemic control but also 1 hour and 2 hour values of postprandial plasma glucose and early morning fasting plasma glucose level can be improved. As an amount of a single dose, 5 to 22 mg is preferable, using 10 to 11 mg of mitiglinide calcium salt hydrate is more preferable. An administration method is administration basically before meal (within 10 minutes before starting meal) or preferably just before meal (within 5 minutes before starting meal), three times a day, and treatment period is preferably 4 weeks or more. In addition, the most preferable is administrating 10 to 11 mg of mitiglinide calcium salt hydrate as a single dose (to be adjusted in consideration of the symptoms) before meal (within 10 minutes before starting meal), preferably just before meal (within 5 minutes before starting meal), tree times a day for 4 weeks or more.

Mitiglinide or pharmaceutically acceptable salts thereof, or hydrates thereof as an active ingredient of the present invention can be easily prepared by the methods described in Japan Patent Publication No.356459/1992 pamphlet, Japan Patent Publication No.340622/1994 pamphlet and Japan Patent Publication No. 340623/1994 pamphlet or similar methods thereto.

As pharmaceutical compositions used in the present invention, oral pharmaceutical compositions such as granules, fine granules, powders, tablets, capsules or the like can be illustrated.

These pharmaceutical compositions can be prepared by admixing with appropriate pharmaceutical additives such as diluents, binders, surfactants, lubricants, glidants, coating materials, plasticizers, coloring agents, flavoring agents and the like in a usually used way pharmaceutically, and formulating in accordance with conventional methods.

Diluents can include, for example, cellulose or cellulose derivatives such as microcrystalline cellulose and the like; starch or starch derivatives such as corn starch, wheat starch, cyclodextrin and the like; sugar or sugar alcohol such as lactose, D-mannitol and the like; and inorganic diluents such as dried aluminum hydroxide gel, precipitated calcium carbonate, magnesium aluminometasilicate, dibasic calcium phosphate and the like.

Binders can include, for example, hydroxypropylcellulose, methylcellulose, hydroxypropylmethylcellulose, povidone, dextrin, pullulane, hydroxypropyl starch, polyvinyl alcohol, gum arabic, agar, gelatin, tragacanth, macrogol and the like.

Surfactants can include, for example, sucrose esters of fatty acids, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monoleaurate, polysorbate, glyceryl monostearate, sodium lauryl sulfate, lauromacrogol and the like.

Lubricants can include,for example,stearic acid,calcium stearate, magnesium stearate, talc and the like.

Glidants can include, for example, dried aluminum hydroxide gel, magnesium silicate and the like.

Coating materials can include, for example, hydroxypropylmethylcellulose 2910, aminoalkyl methacrylate copolymer E, polyvinylacetal diethylaminoacetate, macrogol 6000, titanium oxide and the like.

Plasticizers can include, for example, triethyl citrate, triacetin, macrogol 6000 and the like.

Among pharmaceutical compositions of the present invention, an immediate release formulation is preferable, which can be formulated, for example, by the method described in International Patent Publication No.2000/71117 pamphlet or similar methods thereto.

In the pharmaceutical compositions of the present invention, other hypoglycemic drug(s) can be suitably combined (admixed) with mitiglinide or a pharmaceutically acceptable salt thereof, or a hydrate thereof as an active ingredient. Furthermore, the pharmaceutical compositions of the present invention can be suitably used (in combination) with other hypoglycemic drug(s) at the same time or different times. Examples of the drugs which can be used in combination with the compounds of the present invention include an insulin sensitivity enhancer (pioglitazone hydrochloride, rosiglitazone maleate and the like), a glucose absorption inhibitor (voglibose, acarbose, miglitol and the like), a biguanide (metformin hydrochloride, buformin hydrochloride and the like), an insulin secretion enhancer (tolbutamide, acetohexamide, tolazamide, glyclopyramide, glybuzole, glyburide / glibenclamide, gliclazide, glimepiride and the like), and an insulin preparation and the like.

### [Examples]

The present invention is further illustrated in more detail by way of the following Test Examples and Examples. However, the present invention is not limited thereto.

### Example 1

After 275.0 g of microcrystalline cellulose, 279.0 g of lactose, 100.0 g of corn starch, 30.0 g of low substituted hydroxypropylcellulose (brand name: L-HPC/LH-11, produced by Shin-Etsu Chemical Co., Ltd.), 8.0 g of calcium stearate and 8.0 g of light anhydrous silicic acid (brand name: Adsolider™ 101, produced by Freund Industrial Co., Ltd.) were mixed with 50.0 g of mitiglinide calcium salt hydrate, the mixture was compressed by a tabletting machine to prepare tablets of the following composition.

| | |
|---|---|
| Active component | 10.0 mg |
| Microcrystalline cellulose | 55.0 mg |
| Lactose | 55.8 mg |
| Corn starch | 20.0 mg |
| Low substituted hydroxypropylcellulose | 6.0 mg |
| Calcium stearate | 1.6 mg |
| Light anhydrous silicic acid | 1.6 mg |
| [Total] | 150.0 mg |

### Example 2

After 275.0 g of microcrystalline cellulose, 274.0 g of lactose, 100.0 g of corn starch, 30.0 g of low substituted hydroxypropylcellulose (brand name: L-HPC/LH-11; produced by Shin-Etsu Chemical Co., Ltd.), 8.0 g of calcium stearate and 8.0 g of light anhydrous silicic acid (brand name: Adsolider™ 101, produced by Freund Industrial Co., Ltd.) were mixed with 55.0 g of mitiglinide calcium salt hydrate, the mixture was compressed by a tabletting machine to prepare tablets of the following composition.

| | |
|---|---|
| Active component | 11.0 mg |
| Microcrystalline cellulose | 55.0 mg |
| Lactose | 54.8 mg |
| Corn starch | 20.0 mg |
| Low substituted hydroxypropylcellulose | 6.0 mg |
| Calcium stearate | 1.6 mg |
| Light anhydrous silicic acid | 1.6 mg |
| [Total] | 150.0 mg |

### Test Example 1

### Dissolution test

For the following tablets, the dissolution test was carried out using 900 mL of the first fluid of the Japanese Pharmacopoeia as a test solution and at 50 rpm, according to the paddle method, apparatus 2 of the dissolution test methods of the 13th revised Japanese Pharmacopoeia.

**[Table 1]**

| Tablet | % of dissolution at 20 min after the beginning of the test |
|---|---|
| Example 1 | >75 |
| Example 2 | >75 |

The results on the percentages of dissolution at 20 min after the beginning of the test are shown in Table 1. It has been confirmed that the dissolution time for 75% release in the first fluid of the Japanese Pharmacopoeia of these tablets of Examples 1 and 2 are not more than 20 minutes.

### Example 3

### Clinical study in type II diabetic patients

Using the pharmaceutical composition described in Example 1, a clinical study was conducted in type II diabetic patients under the following conditions.

Inclusion criteria: a type II diabetic patient who did not achieve sufficient glycemic control with diet therapy, more particularly, who has been put on diet therapy since more than 8 weeks before the start of the test drug administration, but the both results of the twice HbA_{1C} measurement are not less than 6.5%, and the 1 hour or 2 hour value of postprandial plasma glucose (PPG) is not less than 200 mg/dL.

Test drug and Mode of administration: Every patient orally administered either of a combination selected from the following combination groups (one tablet from each) three times a day just before meals (within 5 minutes before starting meal):

| | |
|---|---|
| The present invention group | (1) + (4) |
| Positive control group | (2) + (3) |
| Positive control group | (3) + (4) |

(1) a tablet comprising 10 mg of mitiglinide calcium salt hydrate;
(2) a tablet comprising 0.2 mg of voglibose (chemical name: (+)-1L-[1(OH),2,4,5/3]-5-[2-hydroxy-1-(hydroxymethyl)-ethyl]amino-1-*C*-(hydroxymethyl)-1,2,3,4-cyclohexaneterol);
(3) a placebo tablet of mitiglinide calcium salt hydrate not containing any active ingredient; and
(4) a placebo tablet of voglibose not containing any active ingredient.

Treatment period: 12 weeks

Observation item: The following items were measured before administration, at a certain fixed time after the start of administration and at the completion of administration, and their changes were calculated, or the frequency of adverse drug reactions was calculated, and then evaluated.

### [1] Change in HbA_{1C} value

**[Table 2]**

| Treatment Group | Mean value(%) | | | |
|---|---|---|---|---|
| | 4 weeks | 8 weeks | 12 weeks | The final evaluation |
| The present invention group | -0.30 | -0.46 | -0.46 | -0.44 |
| Positive control group | -0.14 | -0.14 | -0.11 | -0.11 |
| Placebo group | 0.02 | 0.14 | 0.22 | 0.21 |

The result on the changes in HbA_{1C} value is shown in the above Table 2. Mitiglinide calcium salt hydrate significantly lowered HbA_{1C} value after 4 weeks after the start of administration in comparison with voglibose as the positive control and placebo, and voglibose significantly lowered HbA_{1C} value in comparison with placebo. From the results mentioned above, it has been confirmed that mitiglinide calcium salt hydrate shows a potent activity lowering HbA_{1C} value and has excellent efficacy to improve glycemic control state.

### [2] Change in early morning fasting plasma glucose (FPG)

**[Table 3]**

| Treatment Group | Mean (mg/dL) |
|---|---|
| The present invention group | -8.0 |
| Positive control group | 0.5 |
| Placebo group | 7.1 |

The result on the changes in early morning fasting plasma glucose (FPG) is shown in the above Table 3 (the mean values in the table indicate values at the final evaluation). Mitiglinide calcium salt hydrate significantly lowered early morning fasting plasma glucose (FPG) in comparison with voglibose as the positive control and placebo. Therefore, it has been confirmed that mitiglinide calcium salt hydrate shows a potent activity to lower early morning fasting plasma glucose (FPG).

### [3] Change in 1 hour and 2 hour values of postprandial plasma glucose (PPG)

**[Table 4]**

| Treatment Group | Mean value(mg/dL) | |
|---|---|---|
| | 1 hour value of PPG | 2 hour value of PPG |
| The present invention group | -53.1 | -50.1 |
| Positive control group | -24.8 | -5.1 |
| Placebo group | 7.1 | 9.9 |

The result on the changes in 1 hour and 2 hour values of postprandial plasma glucose (PPG) is shown in the above Table 4 (the mean values in the table indicate values at the final evaluation). Mitiglinide calcium salt hydrate significantly lowered 1 hour and 2 hour values of postprandial plasma glucose (PPG) in comparison with voglibose as the positive control and placebo. Therefore, it has been confirmed that mitiglinide calcium salt hydrate shows a potent activity to lower postprandial plasma glucose (PPG).

### [4] Frequency of adverse drug reactions of hypoglycemic symptoms and gastrointestinal disorder

**[Table 5]**

| Treatment Group | Frequency(%) | |
|---|---|---|
| | Hypoglycemic symptoms | Gastrointestinal disorder |
| The present invention group | 2.0 | 17.6 |
| Positive control group | 4.5 | 24.5 |
| Placebo group | 2.9 | 16.7 |

The result on the frequencies of adverse drug reactions of hypoglycemic symptoms and gastrointestinal disorders is shown in the above Table 5. Mitiglinide calcium salt hydrate decreased the frequencies of hypoglycemic symptoms and gastrointestinal disorders such as an increase of abdominal wind in comparison with voglibose as the positive control. Therefore, it has been confirmed that mitiglinide calcium salt hydrate is a highly safe agent with low incidences of those adverse drug reactions.

### Example 4

### Clinical study on administration timing

Administration timing before taking a meal was evaluated in healthy male adults. Used test drugs were a tablet comprising 10 mg of mitiglinide calcium salt hydrate and a placebo tablet. The tablet comprising 10 mg of mitiglinide calcium salt hydrate was administered at 0.5 min, 5 min, 10 min or 30 min before starting meal (Positive group), while the placebo tablet was administered at 0.5 min before starting meal. After administration, blood glucose levels were measured at starting meal and evaluated.

**[Table 6]**

| Treatment Group | Administration timing | Mean (mg/dL) |
|---|---|---|
| Positive group | 0.5 min before starting meal | 87.0 |
| | 5 min before starting meal | 83.8 |
| | 10 min before starting meal | 84.2 |
| | 30 min before starting meal | 55.7 |
| Placebo group | 0.5 min before starting meal | 85.6 |

The result is shown in the above Table 6. Mitiglinide calcium salt hydrate was able to maintain good blood glucose level when administrated within 10 minutes before starting meal, while the blood glucose level notably declined when it was administered at 30 minutes before starting meal. Therefore, it has been confirmed that the risk of hypoglycemia incidence can be reduced by administrating mitiglinide calcium salt hydrate within 10 minutes before starting meal. In addition, administration within 5 minutes before starting meal was preferable from the point of view of the administration compliance.

### Industrial Applicability

The present invention can provide a clinically effective, excellent pharmaceutical composition for prevention or treatment of type II diabetes, which can achieve good state of glycemic control and correct postprandial hyperglycemia and early morning fasting hyperglycemia, further, with low frequency of adverse drug reactions such as hypoglycemic symptoms and gastrointestinal disorders.

## Claims

1. A pharmaceutical composition for glycemic control of a type II diabetic patient, which is prepared for administration before meal and comprises mitiglinide or a pharmaceutically acceptable salt thereof, or a hydrate thereof, wherein the amount to be administered as a single dose is 5 to 45 mg.

2. A pharmaceutical composition as claimed in claim 1 wherein the composition is prepared for administration just before meal.

3. A pharmaceutical composition as claimed in claim 1 or 2 wherein the composition is to be administered three times a day before each meal for 4 weeks or more.

4. A pharmaceutical composition as claimed in any of claims 1 to 3 wherein the amount to be administered as a single dose is 5 to 22 mg.

5. A pharmaceutical composition as claimed in claim 4 wherein the amount to be administered as a single dose is 10 to 11 mg and the active ingredient is mitiglinide calcium salt hydrate.

6. A pharmaceutical composition as claimed in any of claims 1 to 5 wherein the dissolution time for 75% release in the first fluid of the Japanese Pharmacopoeia is 20 minutes or less.

7. A pharmaceutical composition as claimed in any of claims 1 to 6 wherein the composition is an agent for prevention or treatment of type II diabetes.

8. A pharmaceutical composition as claimed in claim 7 wherein the type II diabetes is a postprandial hyperglycemia.

9. A use of a pharmaceutical composition as claimed in any of claims 1 to 6 for glycemic control of a type II diabetic patient.

10. A use of a pharmaceutical composition as claimed in any of claims 1 to 7 for prevention or treatment of type II diabetes.

11. A use as claimed in claim 10 wherein the type II diabetes is a postprandial hyperglycemia.

12. A method for glycemic control of a type II diabetic patient, which comprises administrating before meal 5 to 45 mg of mitiglinide or a pharmaceutically acceptable salt thereof, or a hydrate thereof as a single dose.

13. A method for glycemic control as claimed in claim 12 wherein the single dose is 5 to 22 mg.

14. A method for glycemic control as claimed in claim 13 wherein the single dose is 10 to 11 mg and the active ingredient is mitiglinide calcium salt hydrate.

15. A method for glycemic control as claimed in any of claims 12 to 14 wherein the type II diabetes is a postprandial hyperglycemia.

16. A method for improvement of postprandial hyperglycemia of a type II diabetic patient, which comprises administrating before meal 5 to 45 mg of mitiglinide or a pharmaceutically acceptable salt thereof, or a hydrate thereof as a single dose.

17. A method for improvement as claimed in claim 16 wherein the single dose is 5 to 22 mg.

18. A method for improvement as claimed in claim 17 wherein the single dose is 10 to 11 mg and the active ingredient is mitiglinide calcium salt hydrate.

19. A method for improvement as claimed in any of claims 12 to 18 wherein the number of doses a day is three.

20. A method for improvement as claimed in claim 19 wherein the treatment period is 4 weeks or more.

21. A use of mitiglinide or a pharmaceutically acceptable salt thereof, or a hydrate thereof for the manufacture of a pharmaceutical composition as claimed in any claims 1 to 8.
